# EUROPEAN PATENT SPECIFICATION

(11) **EP 3 052 653 B1**
(45) Date of publication and mention of the grant of the patent: **05.12.2018**
(21) Application number: 14792753.7
(22) Date of filing: 02.10.2014
(51) Int. Cl.: C12Q 1/68, C12N 15/10, C12N 15/115

(54) **A METHOD OF IDENTIFYING OR PRODUCING AN APTAMER**
VERFAHREN ZUR IDENTIFIZIERUNG ODER HERSTELLUNG EINES APTAMERS
PROCÉDÉ D'IDENTIFICATION OU DE PRODUCTION D'UN APTAMÈRE

(30) Priority: 02.10.2013 US 201361885519 P
(43) Date of publication of application: 10.08.2016
(73) Proprietor: Tolle, Fabian, 53121 Bonn (DE); Friedrich, Felix, 61184 Karben (DE); Heckel, Alexander, 60438 Frankfurt (DE); Mayer, Günter, 53123 Bonn (DE)
(72) Inventor: Tolle, Fabian, 53121 Bonn (DE); Friedrich, Felix, 61184 Karben (DE); Heckel, Alexander, 60438 Frankfurt (DE); Mayer, Günter, 53123 Bonn (DE)
(74) Representative: Michalski Hüttermann & Partner Patentanwälte mbB
(86) International application number: PCT/EP2014/071205
(87) International publication number: WO 2015/049356

(56) References cited:
- WO-A1-2009/012410
- US-A1- 2011 210 017
- US-A1- 2014 100 120
- YUNFENG CHENG ET AL: "Design, Synthesis, and Polymerase-Catalyzed Incorporation of Click-Modified Boronic Acid-TTP Analogues", CHEMISTRY - AN ASIAN JOURNAL, vol. 6, no. 10, 4 October 2011 (2011-10-04), pages 2747-2752, XP055160640, ISSN: 1861-4728, DOI: 10.1002/asia.201100229
- SAMRA OBEID ET AL: "Interactions of non-polar and "Click-able" nucleotides in the confines of a DNA polymerase active site", CHEMICAL COMMUNICATIONS, vol. 48, no. 67, 1 January 2012 (2012-01-01), page 8320, XP055160951, ISSN: 1359-7345, DOI: 10.1039/c2cc34181f
- GRAMLICH PHILIPP M E ET AL: "Postsynthetic DNA modification through the copper-catalyzed azide-alkyne cycloaddition reaction", ANGEWANDTE CHEMIE INTERNATIONAL EDITION, WILEY - V C H VERLAG GMBH & CO. KGAA, DE, vol. 47, no. 44, 20 October 2008 (2008-10-20), pages 8350-8358, XP009120328, ISSN: 1433-7851, DOI: 10.1002/ANIE.200802077 [retrieved on 2008-09-22]
- PHILIPP M E GRAMLICH ET AL: "Click?Click?Click: Single to Triple Modification of DNA", ANGEWANDTE CHEMIE INTERNATIONAL EDITION, WILEY - V C H VERLAG GMBH & CO. KGAA, DE, vol. 47, no. 18, 1 January 2008 (2008-01-01), pages 3442-3444, XP007916867, ISSN: 1433-7851, DOI: 10.1002/ANIE.200705664 [retrieved on 2008-03-28]
- KEEFE A D ET AL: "SELEX with modified nucleotides", CURRENT OPINION IN CHEMICAL BIOLOGY, CURRENT BIOLOGY LTD, LONDON, GB, vol. 12, no. 4, 1 August 2008 (2008-08-01) , pages 448-456, XP024528065, ISSN: 1367-5931, DOI: 10.1016/J.CBPA.2008.06.028 [retrieved on 2008-07-21]
- MAYER G ET AL: "From selection to caged aptamers: Identification of light-dependent ssDNA aptamers targeting cytohesin", BIOORGANIC & MEDICINAL CHEMISTRY LETTERS, PERGAMON, AMSTERDAM, NL, vol. 19, no. 23, 1 December 2009 (2009-12-01), pages 6561-6564, XP026736055, ISSN: 0960-894X, DOI: 10.1016/J.BMCL.2009.10.032 [retrieved on 2009-10-14]
- CLARA BRIEKE ET AL: "Light-Controlled Tools", ANGEWANDTE CHEMIE INTERNATIONAL EDITION, vol. 51, no. 34, 20 August 2012 (2012-08-20), pages 8446-8476, XP055160738, ISSN: 1433-7851, DOI: 10.1002/anie.201202134
- VARATHARASA THIVIYANATHAN ET AL: "Aptamers and the next generation of diagnostic reagents", PROTEOMICS - CLINICAL APPLICATIONS, vol. 6, no. 11-12, 15 December 2012 (2012-12-15), pages 563-573, XP055160488, ISSN: 1862-8346, DOI: 10.1002/prca.201200042
- FABIAN TOLLE ET AL: "Dressed for success - applying chemistry to modulate aptamer functionality", CHEMICAL SCIENCE, vol. 4, no. 1, 1 January 2013 (2013-01-01) , page 60, XP055160479, ISSN: 2041-6520, DOI: 10.1039/c2sc21510a
- LATHAM J A ET AL: "The application of a modified nucleotide in aptamer selection : novel thrombin aptamers containing 5-(1-pentynyl)-2'-deoxyuridine", NUCLEIC ACIDS RESEARCH, OXFORD UNIVERSITY PRESS, GB, vol. 22, no. 14, 1 January 1994 (1994-01-01), pages 2817-2822, XP002176163, ISSN: 0305-1048
- MOHAMMAD MEHEDI MASUD ET AL: "Sialyllactose-binding modified DNA aptamer bearing additional functionality by SELEX", BIOORGANIC & MEDICINAL CHEMISTRY, vol. 12, no. 5, 1 March 2004 (2004-03-01), pages 1111-1120, XP055160822, ISSN: 0968-0896, DOI: 10.1016/j.bmc.2003.12.009
- KOLB H C ET AL: "The growing impact of click chemistry on drug discovery", DRUG DISCOVERY TODAY, ELSEVIER, RAHWAY, NJ, US, vol. 8, no. 24, 15 December 2003 (2003-12-15), pages 1128-1137, XP002377521, ISSN: 1359-6446, DOI: 10.1016/S1359-6446(03)02933-7

## Description

### FIELD OF THE INVENTION

The present invention relates to the technical fields of techniques for producing oligonucleotides that specifically bind to a target. The present invention relates to a method of identifying or producing an aptamer.

### BACKGROUND OF THE INVENTION

Systematic Evolution of Ligands by Exponential Enrichment (SELEX), also referred to as in vitro selection or in vitro evolution, is a technique for producing oligonucleotides of either single-stranded DNA or RNA that specifically bind to a target ligand. The SELEX method is for example described in document US 5270163. In the SELEX method, a candidate mixture of single stranded nucleic acids having regions of randomized sequence is contacted with a target structure and those nucleic acids having an increased affinity to the target are selected and amplified. After several iterations a nucleic acid with good affinity to the target is obtained.

Philipp M. E. Gramlich et al. describe in Angew. Chem. Int. Ed. 2008, vol 47, no. 18, pages 3442-3444 click-reactions for single to triple modification of DNA. Anthony D Keefe and Sharon T Cload describe in Current Opinion in Chemical Biology, vol. 12, no. 4, 2008, pages 448-456 "SELEX with modified nucleotides".

Many targets cannot be addressed by traditional SELEX approaches. One reason is the limited chemical diversity of nucleic acids. Using chemically modified nucleic acids can solve this issue. However, chemically modified nucleotide-triphosphates often lack compatibility with enzymatic steps, e.g. the polymerase chain reaction (PCR), of the in vitro selection process. Hence, the possible chemical modifications of DNA molecules suitable for in vitro selection are limited. From a practical point of view, use of such modifications is not easily to accomplish since most of them are not commercially accessible and, thus, require laborious chemical synthesis prior use.

Therefore, it is an object of the present invention to provide a method that allows for the introduction of additional chemical entities into DNA during the selection process.

### SUMMARY OF THE INVENTION

It is provided a method of identifying or producing an aptamer comprising the steps of:
(a) Preparing a candidate mixture of nucleic acids, wherein the mixture comprises at least one nucleotide that is modified to comprise a functionalization introduced by click chemistry, wherein the modified nucleotide comprises an alkyne-modified nucleobase that is further modified via a 1,3 dipolar cycloaddition of an azide to yield an azide-alkyne modified nucleobase, wherein the azide comprises a light-sensitive molecule, wherein the light-sensitive molecule is a bulky group inhibiting PCR amplification of the nucleic acid ligand, and wherein the light-sensitive molecule comprises a photo cleavable moiety, wherein upon initiation of cleavage of the photo cleavable moiety by irradiation, a remaining smaller azide-alkyne modified molecule allows amplification;
(b) Contacting the candidate mixture with a target sample;
(c) Partitioning nucleic acids that bind to the target sample from those that do not bind;
(d) Amplifying the binding nucleic acids to produce a population of nucleic acids that bind the target sample, thereby identifying or producing an aptamer.

It has been surprisingly found that the method enables to introduce additional chemical entities into DNA during the selection process. Advantageously, the modification is only transiently present during the selection cycle. This enables a high degree of compatibility with the enzymatic step of the selection process. This approach further allows introducing larger chemical entities into DNA libraries that cannot be used using the methods of the state of the art. The method has a broad acceptance of diverse azide substrates, which are either commercially available or accessible by chemical synthesis.

The method further can comprise displacing one strand of at least one nucleic acid amplified in step (d), and further modifying the nucleic acid to introduce another functionalization.

In accordance with another aspect of the present invention is provided a reagent comprising a nucleic acid ligand capable of binding a target sample, wherein the nucleic acid ligand comprises at least one nucleobase modified to contain an azide-alkyne chemical group, wherein the azide-alkyne chemical group comprises a light-sensitive molecule, wherein the light-sensitive molecule is a bulky group inhibiting PCR amplification of the nucleic acid ligand, and wherein the light-sensitive molecule comprises a photo cleavable moiety, wherein upon initiation of cleavage of the photo cleavable moiety by irradiation, a remaining smaller azide-alkyne modified molecule allows amplification.

Other features and advantages of the present invention will be apparent from the detailed description of the invention and from the claims.

### BRIEF DESCRIPTION OF THE DRAWINGS

Figure 1 shows a Cu(I) catalyzed 1,3-dipolar cycloaddition of an azide with a 5-C8-alkyne modified deoxythymidine.
Figure 2 shows a schematic representation of an embodiment of the method.
Figure 3 shows an embodiment of a light-sensitive modification of an azide-alkyne chemical group.
Figure 4 shows a diagram of the results of an embodiment of the method targeting the MAP kinase ERK and employing a light-sensitive NPE Indole modification.

Interaction profile of a starting library (white bars) and the enriched libraries obtained from selection cycles 8 (grey bars) and 10 (black bars). DNA molecules were radioactively labeled with 32P at 5'-end and incubated with Erk coupled to magnetic particles. As control, non-derivatized magnetic particles were used (empty beads). No binding of the starting or round 10 libraries was detected to empty beads. In turn, round 10 showed a stronger binding to the Erk-beads compared to the starting library, but only its clicked version. This is reflected by the higher amount of DNA that could be eluted from the beads. Photo-cleavage of the modification (10th cycle cleaved) resulted in a loss of binding.

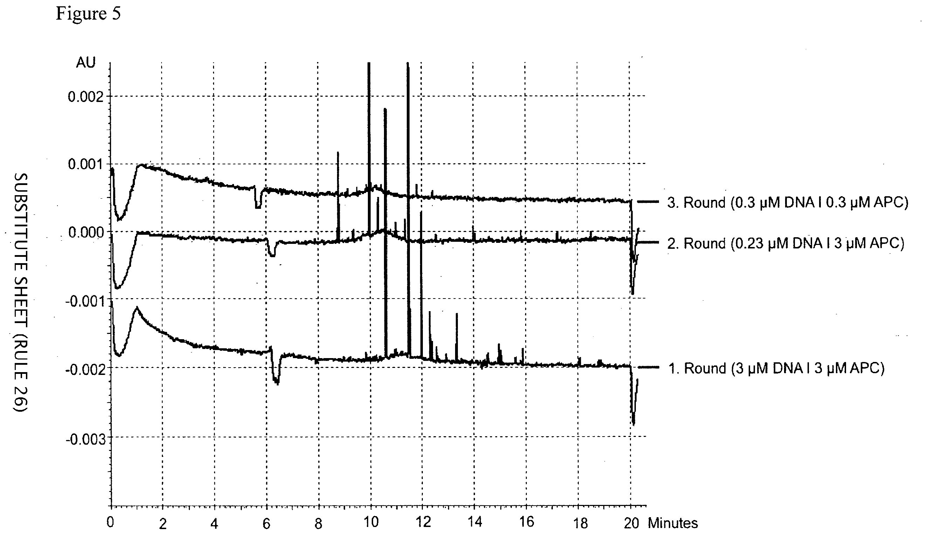

Figure 5 shows an electropherogram of a representative run of rounds 1 to 3 according to an embodiment of the method targeting the Human Activated Protein C (APC). Although the DNA and APC concentration were reduced by a factor of 10 respectively the peak intensity representing the APC/DNA complex did not decrease, indicating an enrichment of DNA sequences with a stronger binding affinity to APC.

### DEFINITIONS

As used herein, the term "aptamer" refers to a small single-stranded oligonucleotide that recognises its target with high specificity and binds to the target with high affinity.

As used herein, the term "alkyne" refers to an unsaturated carbon moiety that has a carbon carbon triple bond between two carbon atoms.

As used herein, the term "azide" refers to a functional group RN₃. The group R refers to a chemical modification or additional chemical entity that can be introduced into the DNA. For example the group R can be a bulky photo cleavable chemical entity.

As used herein, the term "azide-alkyne chemical group" refers to a reaction product of an alkyne and an azide. The reaction can be an 1,3-dipolar cycloaddition of the azide with the alkyne. Via 1,3-dipolar cycloaddition of an azide with an alkyne the azide-alkyne linkeage can be a triazole linkage.

As used herein, the term "click chemistry" refers to bio-orthogonal reactions known to those of ordinary skill in the art. The term "click chemistry" can refer to a 1,3-dipolar cycloaddition. An example of click chemistry is the 1,3-dipolar cycloaddition of an azide to an alkyne, for example using the Cu(I) catalyzed 1,3-dipolar cycloaddition of an azides with an alkyne (CuAAC), or the copper-free strain-promoted azide-alkyne cycloaddition (SPAAC).

As used herein, the term "candidate mixture" refers to a mixture of nucleic acids of differing sequence, from which to select a desired ligand. The source of a candidate mixture can be from naturally-occurring nucleic acids or fragments thereof, chemically synthesized nucleic acids, enzymatically synthesized nucleic acids or nucleic acids made by a combination of the foregoing techniques.

As used herein, the term "ligand" refers to a nucleic acid that binds another molecule, e.g. the target. In a mixture of candidate nucleic acids, a ligand binds with greater affinity than that of the bulk mixture. In a candidate mixture can be comprised more than one ligand for a given target. The ligands can differ from one another in their binding affinities for the target.

As used herein, the term "nucleobase" refers to the nitrogen-containing biological compounds found within the DNA and RNA nucleotides and nucleotides, usually referred to as bases. The primary nucleobases are cytosine (C), guanine (G), adenine (A), thymine (T), and uracil (U).

As used herein, the term "nucleotide" refers to the basic building blocks of nucleic acis comprising or consisting of a nucleobase bound, ribose or deoxyribose and at least one phosphate group.

If not stated otherwise, the term "nucleic acid" refers to a ribonucleic acid or a deoxyribonucleic acid, single-stranded or double stranded. The aptamer according to the invention hence can be provided in the form of a single-stranded DNA or RNA molecule.

As used herein, the term "target sample" refers to any compound of interest for which an aptamer is desired. A sample can be any material, which probably contains a target to be determined, including any liquid or fluid sample or solid material, including a sample derived from a biological source. The term sample refers to polypeptides, peptides, or small organic molecules. The term sample also refers to biological material, for example cells, cell organelles, or tissues, or extracts of any of the foregoing, biological fluids, biological molecules, or supernatants.

As used herein, the term "amplifying" refers to any process or combination of process steps that increases the amount or number of copies of a molecule such as a nucleic acid. Polymerase chain reaction (PCR) is an exemplary method for amplifying of nucleic acids. As used herein, the term "partitioning" refers to any process whereby apatamers bound to the target sample can be separated from nucleic acids not bound to the target. Partitioning for example can be accomplished by immobilization of the nucleic acids bound to the target on magnetic beads or by capillary electrophoresis of the bound nucleic acids against the target. The choice of partitioning method will depend on properties of the target sample and of the nucleic acids that bind to the target sample and can be made according to principles and properties known to those of ordinary skill in the art.

Unless defined otherwise, the technical and scientific terms used herein have the same meaning as commonly understood by one of ordinary skill in the art to which the invention belongs.

It should be noted that, as used in this specification and the appended claims, the singular forms "a", "an" and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

### DETAILED DESCRIPTION OF THE INVENTION

According to the invention it is provided a method of identifying or producing an aptamer comprising the steps of:
(a) Preparing a candidate mixture of nucleic acids, wherein the mixture comprises at least one nucleotide that is modified to comprise a functionalization introduced by click chemistry, wherein the modified nucleotide comprises an alkyne-modified nucleobase that is further modified via a 1,3 dipolar cycloaddition of an azide to yield an azide-alkyne modified nucleobase, wherein the azide comprises a light-sensitive molecule, wherein the light-sensitive molecule is a bulky group inhibiting PCR amplification of the nucleic acid ligand, and wherein the light-sensitive molecule comprises a photo cleavable moiety, wherein upon initiation of cleavage of the photo cleavable moiety by irradiation, a remaining smaller azide-alkyne modified molecule allows amplification;
(b) Contacting the candidate mixture with a target sample;
(c) Partitioning nucleic acids that bind to the target sample from those that do not bind;
(d) Amplifying the binding nucleic acids to produce a population of nucleic acids that bind the target sample, thereby identifying or producing an aptamer.

The method enables the introduction of additional chemical entities into DNA during the selection process. Advantageously, the modification is only transiently present during the selection cycle. This enables a high degree of compatibility with the enzymatic step of the selection process. This approach further allows introducing larger chemical entities into DNA libraries that cannot be used using known methods. The method has a broad acceptance of diverse azide substrates, which are either commercially available or accessible by chemical synthesis.

In an embodiment, the method further comprises displacing one strand of at least one nucleic acid amplified in step (d), and further modifying the nucleic acid to introduce another functionalization.

A DNA library modified to comprise a functionalization, e.g. an azide-alkyne chemical group, can be incubated with a target molecule. After incubation, the unbound sequences will be removed and the bound molecules recovered and amplified by PCR. The method of displacing one strand of the nucleic acid can be made according to principles and properties known to those of ordinary skill in the art. Single-stranded DNA can be achieved by selective lambda exonuclease digestion of one strand. The resultant DNA then again can be modified by re-introducing the chemical modification. This library again can be subjected to an in vitro selection cycle until an enrichment of target-binding sequences can be detected.

In a first SELEX cycle, in step (a) a candidate mixture of nucleic acids can be provided that is prepared by chemical synthesis. When displacing one strand of at least one nucleic acid amplified in step (d), and further modifying the nucleic acid to introduce another functionalization, e.g. an azide-alkyne chemical group, in the following cycles the candidate mixtures can be prepared in a semi enzymatic way.

Particularly click chemistry on the basis of 1,3 dipolar cycloadditions is usable in aqueous biological systems to introduce chemical entities. Several types of reaction have been identified that are usable for click chemistry via 1,3 dipolar cycloaddition. Particularly useful has proven the 1,3 dipolar cycloaddition of an azide to an alkyne. Further, the modification of the nucleobase has proven useful in the method. Hence, in an embodiment, the candidate mixture comprises at least one nucleobase that is modified to comprise an azide-alkyne chemical group.

In an embodiment, the amplification step (d) employs a polymerase chain reaction using an alkyne-modified nucleobase that is further modified via 1,3 dipolar cycloaddition of an azide to yield an azide-alkyne modified nucleobase.

The nucleic acids of the candidate mixture can be single-stranded DNA. The introduction of additional chemical entities into DNA during the selection process can be achieved by using a 5-alkyne modified nucleobase, for example thymine. 5-C8-alkyne modified nucleotide-triphosphates for example deoxythymidines are commercially available or can be synthesized. Such 5-C8-alkyne modified nucleobases for example deoxythymidines can be introduced into DNA by PCR. The modification can be further derivatized with so called bio-orthogonal chemistry, for example using the Cu(I) catalyzed 1,3-dipolar cycloaddition of respective azides with the alkyne. Beside the Cu(I) catalysed azide-alkyne cycloaddition (CuAAC), copper-free strain-promoted azide-alkyne cycloaddition (SPAAC) reactions also are usable. In applications in cellular or living systems the strain-promoted azide-alkyne cycloaddition can overcome toxicity issues associated with the use of Cu(I).

DNA libraries that are modified in this way can then be employed for in vitro selection purposes. Hence, this reaction enables to introduce chemical modifications into DNA applicable in SELEX. Thereby, the chemical diversity of DNA libraries can be enhanced.

In the present invention, the azide is modified with a light-sensitive molecule. The use of photosensitive modifications further enhances the approach towards bulky residues to be introduced and used in the selection process. It also enables the direct selection of photoresponsive aptamers. This allows the development of entirely new selection protocols.

The light-sensitive molecule is a bulky group that inhibits amplification by PCR. It further comprises a photo cleavable moiety, and cleavage of the bulky group can be initiated by irradiation, for example with ultraviolet light, and the remaining smaller azide-alkyne modified molecule can become available to PCR.

The method can be applied to a broad variety of target samples. This allows for a wide diversity of samples including biological structures. In an embodiment, the target sample is selected from the group consisting of a polypeptide, peptide, cell, cell fragment, cell membrane, small organic molecule, biological fluid sample and combination thereof. Especially, the method is not limited to purified samples, of polypeptides, peptides or small organic molecules, but extendable to highly complex biological contexts such as biological fluid sample or even directly at the membranes of cells.

Another aspect, the invention relates to a reagent comprising a nucleic acid ligand capable of binding a target sample, wherein the nucleic acid ligand comprises at least one nucleobase modified to contain an azide-alkyne chemical group, wherein the azide-alkyne chemical group comprises a light-sensitive molecule, wherein the light-sensitive molecule is a bulky group inhibiting PCR amplification of the nucleic acid ligand, and wherein the light-sensitive molecule comprises a photo cleavable moiety, wherein upon initiation of cleavage of the photo cleavable moiety by irradiation, a remaining smaller azide-alkyne modified molecule allows amplification.

The modified nucleobase for example can be the thymine. The modification can be achieved by using a 5-C8-alkyne modified deoxythymidine that can be introduced into DNA by PCR. The modification can be further derivatized with socalled bio-orthogonal chemistry, for example using the Cu(I) catalyzed 1,3-dipolar cycloaddition of a respective azides with the alkyne.

In the present invention, the azide-alkyne chemical group comprises a light-sensitive molecule. The light-sensitive molecule is a bulky group that inhibits PCR amplification of the nucleic acid ligand. Upon initiation of cleavage of a photo cleavable moiety by irradiation with ultraviolet light, a remaining smaller azide-alkyne modified molecule allows amplification. The invention will be explained in more detail by virtue of the examples set forth herein below. While at least one exemplary embodiment is presented, it should be appreciated that a vast number of variations exist. It should also be appreciated that the exemplary embodiments are only examples, and are not intended to limit the scope, applicability, or configuration in any way. Rather, the forgoing description will provide those with ordinary skill in the art with the essential characteristics of this invention for implementing at least one exemplary embodiment, it being understood that various changes may be made without departing from the scope as set forth in the appended claims.

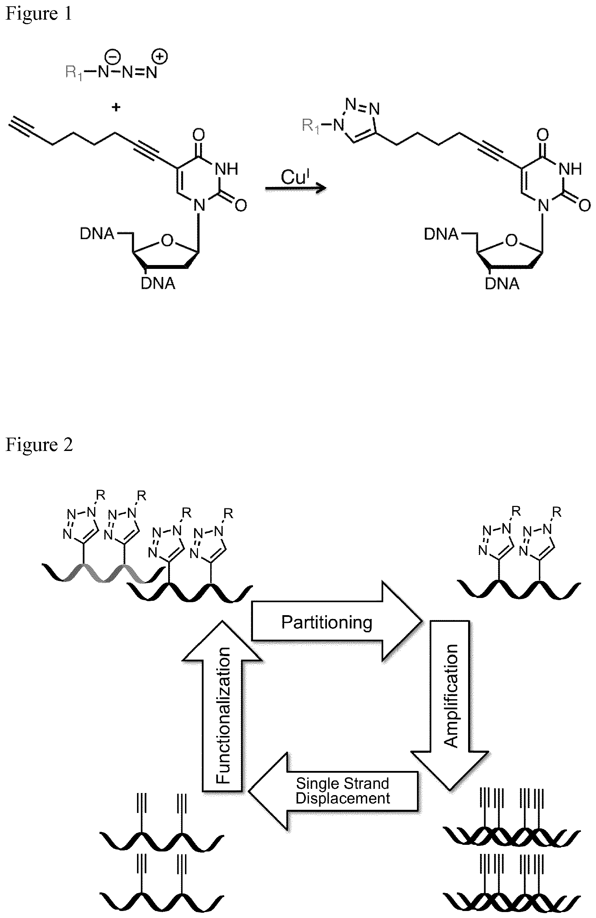

The figure 1 shows a 5-C8-alkyne modified deoxythymidine that is introduced into DNA by PCR. This modification can be further derivatized using Cu(I) catalyzed 1,3-dipolar cycloaddition of an azide R¹N₃.

The figure 2 shows a schematic representation of an embodiment of the method. A DNA library modified to comprise an azide-alkyne group is incubated with a target molecule. After incubation, the unbound sequences are removed and the bound molecules recovered. The selected molecule is amplified by PCR, using an alkyne-modified nucleobase. Single-strand displacement is achieved by selective lambda exonuclease digestion of one strand. The resultant DNA is then again modified by 1,3-dipolar cycloaddition of an azide RN₃, thereby re-introducing the chemical modification of an azide-alkyne group. This library is again subjected to an in vitro selection cycle until an enrichment of target-binding sequences can be detected.

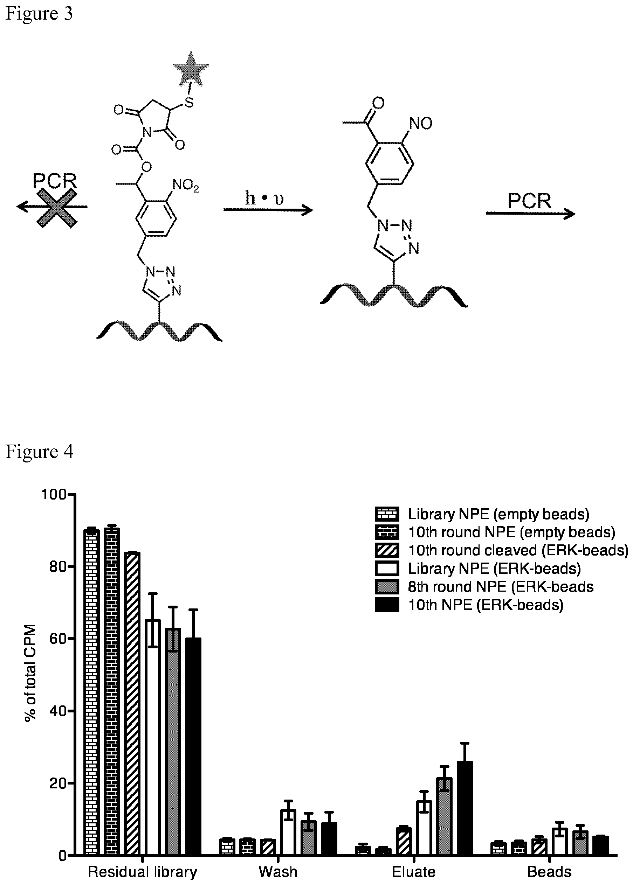

The figure 3 shows a light-sensitive modification. The azide-alkyne group has a light-sensitive molecule that is introduced via the group R of the azide. The bulky molecule inhibits amplification by PCR. Irradiation with ultraviolet light cleaves a photo cleavable moiety, and the remaining molecule allows amplification by PCR.

### EXAMPLES

### Material

**Nucleic Acids:**

| name | specification | supplier |
|---|---|---|
| Library (FT2-N42): CAC GAC GCA AGG GAC CAC AGG NNN NNN NNN NNN NNN NNN NNN NNN NNN NNN NNN NNN NNN NNN CAG CAC GAC ACC GCA GAG GCA; (SEQ ID NO: 1) | | ATDBio, UK |
| N: dA, dC, dG, C8-Alkyne-dU (1:1:1:1) | | |
| CAC GAC GCA AGG GAC CAC AGG (SEQ ID NO: 2) | forward primer (FT2-F) | Microsynth AG, Switzerland |
| Phosphate-TGC CTC TGC GGT GTC GTG CTG (SEQ ID NO: 3) | reverse primer (FT2-R-P) | Microsynth AG, Switzerland |

**Proteins:**

| name | specification | supplier | Catalogue no. |
|---|---|---|---|
| *Pwo* DNA polymerase | 2.5 U / µl | Genaxxon, Germany | M3002.0100 |
| λ-Exonuclease | 10 U / µl | Thermo Scientific | EN0562 |
| Human Activated Protein | | Haematologic | HCAPC-080; Lot: |
| C (APC) | | Technologies | CC0405-1.0MG |
| Bovine Serum Albumine (BSA) | | Sigma-Aldrich | A9418-10G |
| T4 Polynucleotide Kinase | | New England Biolabs | M0201L |

**Chemicals:**

| name | specification | supplier | Catalogue no. |
|---|---|---|---|
| Tris(4-(3-hydroxy-propoyl)-[1,2,3]triazol-1-ylmethyl)amine (THPTA) | | Baseclick, Germany | BCMI-006-5 |
| Copper sulphate | | Sigma-Aldrich | 451657-10G |
| 5-(Octa-1,7-diynyl)-5'-*O-*triphosphate-2'-deoxyuridine (Alkyne-dUTP) | | Baseclick, Germany | BCT-05-L |
| dNTPs | | Larova, Germany | 250 µM |
| Dulbecco's Phosphate Buffered Saline (D-PBS) | | Sigma-Aldrich | D8662-1L |
| Salmon Sperm DNA | | Life Technologies | AM9680 |
| TWEEN® 20 | | Merck Millipore, Germany | 8170721000 |
| ATP [γ-³²P]- 3000Ci/mmol 10mCi/ml | | Perkin Elmer | BLU002A500UC |

**Miscellaneous:**

| name | supplier | Catalogue no. |
|---|---|---|
| Dynabeads® His-Tag Isolation & Pulldown | Life Technologies | 10103D |
| DynaMag™-2 magnetic rack | Life Technologies | 12321D |
| NucleoSpin® Gel and PCR Clean-up | Macherey-Nagel, Germany | 740609.250 |

**Buffers:**

| name | specification |
|---|---|
| SELEX-Buffer | 1x D-PBS + 0.1 % BSA + 0.1 mg/ml salmon sperm + 0.01% Tween20 |
| B15-Buffer | 25 mM Tris, 30 mM NaCl, 1 mM KCl, 1 mM CaCl₂, 1 mM MgCl₂; pH 8.3 |

### Example 1

### Synthesis of an azide modified with a light-sensitive molecule: 1-[5-(Azidomethyl)-2-nitrophenyl]ethyl-[2-(1H-indol-3-yl)ethyl]carbamate

### Step 1.1 - Synthesis of 2-Nitro-5-methyl benzoyl chloride

25 g (0.14 mol) 2-Nitro-5-methylbenzoic acide were dissolved in 200 mL (2.75 mol) of thionyl chloride in a baked flask. A catalytic amount of dimethyl formamide was added and the solution was heated under reflux for 3 h under inert gas. Excess thionyl chloride was removed by distillation. The product was quantitatively isolated as a brown solid and further used without characterisation.

### Step 1.2 - Synthesis of 2-Nitro-5-methyl acetophenone

3.5 g (0.14 mol) magnesium, 0.33 mL (3.4 mmol) carbon tetrachloride, and 3.25 mL (56 mmol) ethanol were heated under Argon atmosphere in a baked flask. After start of the reaction, 130 mL dry ether were added drop-wise. A solution of 22.0 ml (0.14 mol) diethyl malonate in 16.6 mL ether, and additional 13.3 mL (0.23 mol) of ethanol were added. Subsequently, the reaction was heated under reflux until the magnesium was consumed. Afterwards, a mixture of 26 g (0.13 mol) 2-nitro-5-methylbenzoyl chloride of step 1.1 in 32.5 mL dry ether was added. The viscosity of the solution increased. After the stirring stopped 9 mL of sulphuric acid in 130 mL water were added. The resulting phases were separated, and the aqueous phase was washed with ether. The combined organic phases were dried with magnesium sulphate, and the solvent was evaporated. Diethyl-5-methyl-2-nitrobenzoyl malonate was obtained as the intermediate product, and dissolved in 26 mL glacial acetic acid. 5 mL of sulfuric acid was added. The solution was heated under reflux as long as gas formation was seen. Afterwards, the solution was put on ice and alkalized using sodium hydroxide solution. The aqueous phase was extracted with ether. The organic phases was dried with magnesium sulfate, the solvent evaporated, and the raw product was separated by column chromatography using a 15:1 mixture of cyclohexane:ethylacetate. 18.1 g (77 %) of the product were isolated.

### Step 1.3 - Synthesis of 5-(Bromomethyl)-2-nitroacetophenone

18.1 g (0.10 mol) 2-Nitro-5-methyl acetophenone of step 1.2 were dissolved with 18.9 g (0.11 mol) *N*-brom succinimide and 2.4 g (10 mmol) dibenzoyl peroxide in 60 mL carbon tetra chloride and heated under reflux for 16 h. Afterwards, 1.21 g (5 mmol) dibenzoyl peroxide were added and the solution was heated under reflux for another 8 h. The reaction solution was then filtered and the filtrate was concentrated under vacuum. The residue was dissolved in ethyl acetate and extracted with saturated sodium hydrogen solution and saline solution. The organic phase was dried with magnesium sulfate, the solvent evaporated, and the product was cleaned by column chromatography using a 15:1 mixture of cyclohexane : ethyl acetate. The isolated product was dissolved in 200 mL ether and overlaid with 50 mL hexane for crystallization to remove contamination with twofold halogenated product and educt. The solid was filtrated and dried under vacuum. 9.9 g (41 %) of the product were isolated.

### Step 1.4 - Synthesis of 5-Azidomethyl-2-nitroacetophenone

7.76 g (30.2 mmol) 5-(Bromomethyl)-2-nitroacetophenone of step 1.3 were dissolved in 84 mL acetone and 16 mL water, and 2.95 g (45.3 mmol) sodium azide were added. The reaction was heated to 75°C and the reaction process was monitored by thin layer chromatography. After the reaction was completed, the acetone was removed under vacuum and the product was extracted with ethyl acetate. The organic phase was washed with saline solution, dried with magnesium sulphate, the solvent was evaporated, and the residue cleaned with column chromatography using a gradient of cyclohexane:ethylacetate from 10:1 to 5:1. 4.6 g (69 %) of the desired product were isolated.

### Step 1.5 - Synthesis of 1-[5-(Azidomethyl)-2-nitrophenyl]ethanol

4.0 g (18.2 mmol) 5-Azidomethyl-2-nitro acetophenone of step 1.4 were dissolved in a mixture of 60 mL dry methanol and 40 mL dry dioxane. 1.0 g (27.2 mmol) Sodium borohydride was added and the reaction mixture stirred under inert gar atmosphere. The reaction process was monitored by thin layer chromatography. After the reaction was completed, 160 mL water and 6.5 mL 1M hydrochloric acid were added and the product was extracted in ethyl acetate. The organic phase was washed with saturated sodium chloride solution, dried with magnesium sulphate, the solvent was evaporated, and the residue cleaned with column chromatography using a 5:1 mixture of cyclohexane:ethylacetate. 3,66 g (91 %) of the product were isolated.

### Step 1.6 - Synthesis of 1-[5-(Azidomethyl)-2-nitrophenyl]ethyl-N-succinimidyl carbonic acid diester

0.65 g (3.0 mmol) 1-[5-(Azidomethyl)-2-nitrophenyl]ethanol of step 1.5 were dissolved in 6 mL dry acetonitrile. 0.70 mL (5.0 mmol) Triethylamine followed by 1.53 g (6.0 mmol) *N,N'-*discuccinimidyl carbonate were added and the solution was stirred under inert gar atmosphere. The reaction process was monitored by thin layer chromatography. After the reaction was completed, 10 mL water and 0.2 mL saturated sodium chloride solution were added and the product was extracted in ethyl acetate. The organic phase was separated, dried with magnesium sulphate, concentrated under vacuum, and the product finally isolated with column chromatography using a 2:1 mixture of cyclohexane:ethylacetate. 640 mg (80 %) of the product were isolated.

### Step 1.7 - Synthesis of 1-[5-(Azidomethyl)-2-nitrophenyl]ethyl-[2-(1H-indol-3-yl)ethyl] carbamate

640 mg (1,8 mmol) 1-[5-(Azidomethyl)-2-nitrophenyl]ethyl-*N*-succinimidyl carbonic acid diester of step 1.6 were dissolved in 11 mL dry tetrahydrofurane. Subsequently, 0.41 mL (2.9 mmol) triethyl amine and 235 mg (1.47 mmol) tryptamine were added and the solution was stirred for 24 h under inert gas. Afterwards, 55 mL dichlormethane and 170 mL of a 5 % solution of hydrochloric acid were added, and the product was extracted. The aqueous phase was washed twice with dichlormethane, and the combined organic phases were dried with magnesium sulphate. The solvent was removed under vacuum and the residue was cleaned with column chromatography using a gradient of cyclohexane:ethylacetate from 5:1 to 5:3. 520 mg (87 %) of the product were obtained. 30 mg of the product were dissolved in a solution of 60 % acetonitrile and 40 % of 0.1 % trifluoroaceticacid and cleaned via HPLC in ten runs using a 250x4.6 mm Nucleosil 100-5 C18 column. The procedure started with 60 % acetonitrile and 40 % of 0.1 % trifluoroaceticacid and rinsed during 20 minutes to a ratio of 80% : 20%. The retention time of the product 1-[5-(Azidomethyl)-2-nitrophenyl]ethyl-[2-(1*H-*indol-3-yl)ethyl]carbamate was 8.7 minutes.

### Example 2

### Synthesis of 3-(2-Azidoethyl)-1-indole

Under an argon-atmosphere, 0.90 g 3-(2-Bromoethyl)-1H-indole (4.02 mmol) was solved in 10 mL dry DMF and 0.26 g sodium azide (4.02 mmol) was added. After 15 hours at 25 °C the solution was washed sequentially with water. The combined aqueous layers where reextracted with ethylacetate and the combined ethylacetate solutions were dried with MgSO₄ and concentrated. Column chromatography (ethylacetate/petroleum ether 1:9) gave 3-(2-Azidoethyl)-1H-indole as yellow oil. The yield was 0.52 g (70%).

### Example 3

The method targeting the Mitogen-activated Protein Kinase ERK2 and employing a light-sensitive NPE-Indole modification

### 3.1 - Preparing a candidate mixture of nucleic acids

The single stranded DNA starting library comprising 42 wooble positions (FT2-N42: CAC GAC GCA AGG GAC CAC AGG NNN NNN NNN NNN NNN NNN NNN NNN NNN NNN NNN NNN NNN NNN CAG CAC GAC ACC GCA GAG GCA; N: dA, dC, dG, C8-Alkyne-dU (1:1:1:1)) (SEQ ID NO: 1) was synthesized and PAGE purified by ATDBio (Southampton, United Kingdom).

The library was further functionalized with the light sensitive Indol-NPE-Azide 1-[5-(Azidomethyl)-2-nitrophenyl]ethyl-[2-(1*H-*indol-3-yl)ethyl]carbamate prepared according to Example 1 in a Cu(I) catalysed azide-alkyne cycloaddition (CuAAC reaction) as follows: 150 µl of a 3.5 µM DNA solution were mixed with 20 µl D-PBS and 20 µl 10mM azide solution and the reaction was started by addition of 10 µl freshly prepared catalyst solution (10 mM THPTA, 2 mM CuSO₄, 50 mM Sodium ascorbate). The reaction mixture was incubated for 1h at 37 °C, 800 rpm and the functionalized ssDNA was purified with the NucleoSpin® Gel and PCR Clean-up kit according to the manufacturers recommendation.

### 3.2 - Preparation of the target sample

100 µg recombinant expressed and purified His6-tagged ERK2 (His6-ERK2) (kindfully provided by Sabine Lennarz, University of Bonn) were immobilized on 5 mg magnetic beads (Dynabeads His-Tag Isolation) in a total volume of 500 µl SELEX buffer (1x D-PBS + 0.1 % BSA + 0.1 mg/ml salmon sperm + 0.01% Tween20), according to the manufacturers recommendation. The ERK loaded beads were stored at 4°C until further usage.

### 3.3 - Contacting the candidate mixture with the target sample and partitioning the nucleic acids that bind to ERK2 from those that do not bind

100 µl (ca. 500 pmol) clicked and purified DNA start library of step 3.1 in 1X D-PBS were heated to 95°C for 3 min and let slowly cool to ambient temperature afterwards. 50 µl (500 µg) uncoupled magnetic beads (in SELEX buffer) were added and incubated for 30 min at 37 °C, 800 rpm. The supernatant was added to 50 µl (500 µg) ERK2-bead suspension and incubated for 30 min at 37 °C, 800 rpm. The supernatant was discarded and the beads were washed 3 times for 30 sec with 200 µl SELEX buffer. 100 µl 300 mM imidazole-solution were added and incubated for 15 min at 37°C, 800 rpm. This supernatant was used for the subsequent PCR amplification.

### 3.4 - Amplification of the binding nucleic acids

The eluted DNA (enriched library) was divided into 8 aliquots and PCR amplified as follows: All PCR reactions were done in 100 µl reaction volume according to the following pipetting and cycling scheme of table 1 in a Mastercycler® Personal (Eppendorf) thermocycler. The samples were cycled until the desired product band could be detected on an ethidium bromide stained 4% agarose gel.

**Table 1:**

| Reagent | Stock-Conc. | Final-Conc. |
|---|---|---|
| Water | | |
| DNA solution | | |
| PWO-Buffer | 10X | 1X |
| dN*TPs | 25 mM | 250 µM |
| F-Primer | 50 µM | 0.5 µM |
| R-Primer | 50 µM | 0.5 µM |
| PWO-Pol. | 2.5 U/µl | 2.5 U |
| dN*TPs: | dATP / dCTP / dGTP / C8-Alkyne-dUTP | |

The cycling scheme was as follows: 2 min at 95°C, followed by 30 sec at 95 °C / 30 sec at 62 °C / 1 min at 72 °C for X cycles, storage at 4 °C.

The dsDNA PCR products were purified with the NucleoSpin® Gel and PCR Clean-up kit according to the manufacturers recommendation and as a backup of this SELEX round 10 % of the purified dsDNA were stored at -20 °C.

### 3.5 - Single strand displacement

Purified dsDNA PCR product was supplemented with 40 µl 10 X exonuclease reaction buffer and 8 µl lambda exonuclease were added. The mixture was incubate for 60 min at 37 °C, 800 rpm and the ssDNA digestion product was purified with the NucleoSpin® Gel and PCR Clean-up kit according to the manufacturers recommendation

### 3.6 - Functionalization of the enriched library

For the next SELEX round the enriched single stranded library was again further functionalized with the light sensitive Indol-NPE-Azide in a CuAAC reaction as follows: 150 µl of the DNA solution were mixed with 20 µl D-PBS and 20 µl 10mM azide solution and the reaction was started by addition of 10 µl freshly prepared catalyst solution (10 mM THPTA, 2 mM CuSO₄, 50 mM Sodium ascorbate). The reaction mixture was incubated for 1h at 37 °C,

800 rpm and the functionalized ssDNA was purified with the NucleoSpin® Gel and PCR Clean-up kit according to the manufacturers recommendation.

### 3.7 - 2^{nd} to 10^{th} SELEX round

10 µl 10 X D-PBS were added to 90 µl of the enriched clicked DNA library (ca. 50 pmol) and the next SELEX round was performed as described for the first round. In total 10 rounds of SELEX were performed. To increase the selection pressure the amount of ERK2-functionalized beads was reduced and the washing times were increased as summarized in the following table 2:

**Table 2:**

| Round | ERK2-beads [µl] | Wash 1 | Wash 2 | Wash 3 |
|---|---|---|---|---|
| 1 | 50 | 30 sec | 30 sec | 30 sec |
| 2 | 50 | 30 sec | 5 min | 30 sec |
| 3 | 25 | 30 sec | 5 min | 30 sec |
| 4 | 12.5 | 30 sec | 5 min | 30 sec |
| 5 | 5 | 30 sec | 5 min | 30 sec |
| 6 | 2.5 | 30 sec | 5 min | 5 min |
| 7/8/9/10 | 1.25 | 30 sec | 5 min | 5 min |

### 3.8 - Radioactive binding assay

The enriched library of the 8th and 10th SELEX rounds as well as the starting library were radioactive labelled and functionalized with Indole-NPE-Azide as follows: 41 µl purified ssDNA were mixed with 5 µl 10 X T4 PNK reaction buffer, 2 µl T4 PNK and 2 µl γ 32P ATP and the mixture was incubated for 1 h at 37 °C. The radioactive labelled DNA was purified with the NucleoSpin® Gel and PCR Clean-up kit according to the manufacturers recommendation. As a control one sample of the clicked 10th round library was irradiated with UV-light (360 nm) for 5 min in order to cleave the photo sensitive NPE moiety.

A SELEX round (without negative selection) was simulated collecting all fractions (residual library / Wash 1/2/3 / Eluate / Beads). All samples were filled to 1 ml with water and the radioactivity (Cherenkov radiation) was measured for 3 min in a scintillation counter (winSpectral 1414; Perkin Elmer).

The Figure 4 shows a diagram of the results targeting the MAP kinase ERK. The Figure 4 shows the interaction profile of a starting library, given as white bars, and the enriched libraries obtained from selection cycles 8, given as grey bars, and 10, given as black bars. As control, non-derivatized magnetic particles were used (empty beads). As can be taken from the figure 4, no binding of the starting or round 10 libraries was detected to empty beads. In turn, round 10 showed a stronger binding to the Erk-beads compared to the starting library, but only its via click chemistry modified version. This was reflected by the higher amount of DNA that could be eluted from the beads. Photo-cleavage of the modification (10th cycle cleaved) resulted in a loss of binding. This result demonstrates that nucleic acids that bind to the ERK target were successfully identified.

### Example 4

### The method targeting the Human Activated Protein C (APC)

### 4.1 Preparing a candidate mixture of nucleic acids

The single stranded DNA starting library comprising 42 wooble positions (FT2-N42: CAC GAC GCA AGG GAC CAC AGG NNN NNN NNN NNN NNN NNN NNN NNN NNN NNN NNN NNN NNN NNN CAG CAC GAC ACC GCA GAG GCA; N: dA, dC, dG, C8-Alkyne-dU (1:1:1:1)) (SEQ ID NO: 1) was synthesized and PAGE purified by ATDBio (Southampton, United Kingdom).

The library was further functionalized with the Indole-Azide prepared according to Example 2 in a Cu(I) catalysed azide-alkyne cycloaddition (CuAAC reaction) as follows: 150 µl of a 3.5 µM DNA solution were mixed with 20 µl D-PBS and 20 µl 10mM azide solution and the reaction was started by addition of 10 µl freshly prepared catalyst solution (10 mM THPTA, 2 mM CuSO4, 50 mM Sodium ascorbate). The reaction mixture was incubated for 1h at 37 °C, 800 rpm and the functionalized ssDNA was purified with the NucleoSpin® Gel and PCR Clean-up kit according to the manufacturers recommendation.

### 4.2 Contacting the candidate mixture with a target sample and partitioning of nucleic acids that bind to APC from those that do not bind by capillary electrophoresis

100 µl (ca. 500 pmol) clicked and purified DNA start library in 1X B15 buffer (25 mM Tris, 30 mM NaCl, 1 mM KCl, 1 mM CaCl2, 1 mM MgCl2; pH 8.3) were heated to 95°C for 3 min and let slowly cool to RT afterwards. The indole-azide clicked starting library (3 µM final concentration) and APC (3 µM final concentration) were incubated for 10 min in B15 buffer and 3 x 20 min separation runs of capillary electrophoresis were performed as follows. The enriched library of the 3 seperation runs was collected in 80 µl B15 buffer for subsequent PCR amplification.

A PA 800 Capillary Electrophoresis system (Beckman Coulter, Inc., Fullerton, CA, USA) with 32 Karat software (Beckman Coulter, Inc., Fullerton, CA, USA) and equipped with a 50 cm long, 50 µm inner diameter capillary having a 40 cm length to detector was used. Before each CE run, the capillary was rinsed with water and B15 buffer for 5 min. The capillary cartridge was maintained at 25 °C and the sample chamber was held at 20 °C. Electric fields of 25 kV were utilized to drive the electrophoresis separations. The samples (47.5 nl) were injected into the capillary, prefilled with the B15 run buffer, by a pressure pulse of 5 s × 4 psi. Samples were monitored using UV detection at 254 nm.

### 4.3 Amplifying the binding nucleic acids

The enriched library received of step 4.2 was PCR amplified as follows: All PCR reactions were done in 100 µl reaction volume according to the following pipetting and cycling scheme of table 3 in a Mastercycler® Personal (Eppendorf) thermocycler. The samples were cycled until the desired product band could be detected on an ethidium bromide stained 4% agarose gel.

**Table 3:**

| Reagent | Stock-Conc. | Final-Conc. |
|---|---|---|
| Water | | |
| DNA solution | | |
| PWO-Buffer | 10X | 1X |
| dN*TPs | 25 mM | 250 µM |
| F-Primer | 50 µM | 0.5 µM |
| R-Primer | 50 µM | 0.5 µM |
| PWO-Pol. | 2.5 U/µl | 2.5 U |
| dN*TPs: | dATP / dCTP / dGTP / C8-Alkyne-dUTP | |

The cycling scheme was as follows: 2 min at 95°C, followed by 30 sec at 95 °C / 30 sec at 62 °C / 1 min at 72 °C for X cycles, and storage at 4 °C.

The dsDNA PCR products were purified with the NucleoSpin® Gel and PCR Clean-up kit according to the manufacturers recommendation. The first PCR product was divided into 16 aliquots and further PCR amplified as described above. As a backup of this SELEX round 10 % of the purified dsDNA were stored at -20 °C.

### 4.4 Single strand displacement

Purified dsDNA PCR product was supplemented with 40 µl 10 X exonuclease reaction buffer and 8 µl lambda exonuclease were added. The mixture was incubate for 60 min at 37 °C, 800 rpm and the ssDNA digestion product was purified with the NucleoSpin® Gel and PCR Clean-up kit according to the manufacturers recommendation

### 4.5 Functionalization of the enriched library

For the next SELEX round the enriched single stranded library was again further functionalized with Indole-Azide in a CuAAC reaction as follows: 150 µl of the DNA solution were mixed with 20 µl D-PBS and 20 µl 10mM azide solution and the reaction was started by addition of 10 µl freshly prepared catalyst solution (10 mM THPTA, 2 mM CuSO₄, 50 mM Sodium ascorbate). The reaction mixture was incubated for 1h at 37 °C, 800 rpm and the functionalized ssDNA was purified with the NucleoSpin® Gel and PCR Clean-up kit according to the manufacturers recommendation.

### 4.6 2^{nd} to 5^{th} SELEX round

In total 5 rounds of SELEX, following the same protocol as in the first round, were performed. To increase the selection pressure the DNA and APC concentration was reduced as summarized in the following table 4:

**Table 4:**

| Round | APC [µM] | DNA [µM] |
|---|---|---|
| 1 | 3 | 3 |
| 2 | 0.23 | 3 |
| 3 | 0.3 | 0.3 |
| 4 | 0.1 | 0.06 |
| 5 | 0.3 | 0.12 |

The Figure 5 shows the electropherogram of a representative run of rounds 1 to 3. As can be taken from the figure 5, although the DNA and APC concentration were reduced by a factor of 10 respectively the peak intensity representing the APC/DNA complex did not decrease, indicating an enrichment of DNA sequences with a stronger binding affinity to APC.

## Claims

1. A method of identifying or producing an aptamer comprising the steps of:
(a) Preparing a candidate mixture of nucleic acids, wherein the mixture comprises at least one nucleotide that is modified to comprise a functionalization introduced by click chemistry, wherein the modified nucleotide comprises an alkyne-modified nucleobase that is further modified via a 1,3 dipolar cycloaddition of an azide to yield an azide-alkyne modified nucleobase, wherein the azide comprises a light-sensitive molecule, wherein the light-sensitive molecule is a bulky group inhibiting PCR amplification of the nucleic acid ligand, and wherein the light-sensitive molecule comprises a photo cleavable moiety, wherein upon initiation of cleavage of the photo cleavable moiety by irradiation, a remaining smaller azide-alkyne modified molecule allows amplification;
(b) Contacting the candidate mixture with a target sample;
(c) Partitioning nucleic acids that bind to the target sample from those that do not bind;
(d) Amplifying the binding nucleic acids to produce a population of nucleic acids that bind the target sample, thereby identifying or producing an aptamer.

2. The method of claim 1 further comprising displacing one strand of at least one nucleic acid amplified in step (d), and further modifying the nucleic acid to introduce another functionalization.

3. The method of claim 1 or 2, wherein the amplification step (d) employs a polymerase chain reaction using an alkyne-modified nucleobase that is further modified via a 1,3 dipolar cycloaddition of an azide to yield an azide-alkyne modified nucleobase.

4. The method of claim 1, wherein the target sample is selected from the group consisting of a polypeptide, peptide, cell, cell fragment, cell membrane, small organic molecule, biological fluid sample and combination thereof.

5. A reagent comprising a nucleic acid ligand capable of binding a target sample, wherein the nucleic acid ligand comprises at least one nucleobase modified to contain an azide-alkyne chemical group, wherein the azide-alkyne chemical group comprises a light-sensitive molecule, wherein the light-sensitive molecule is a bulky group inhibiting PCR amplification of the nucleic acid ligand, and wherein the light-sensitive molecule comprises a photo cleavable moiety, wherein upon initiation of cleavage of the photo cleavable moiety by irradiation, a remaining smaller azide-alkyne modified molecule allows amplification.

## Patentansprüche

1. Verfahren zum Identifizieren oder Erzeugen eines Aptamers, umfassend die Schritte des:
(a) Herstellens einer Kandidatenmischung von Nucleinsäuren, wobei die Mischung mindestens ein Nucleotid umfasst, das modifiziert worden ist, um eine durch Click-Chemie eingeführte Funktionalisierung zu umfassen, wobei das modifizierte Nucleotid eine durch Alkyn modifizierte Nucleobase umfasst, die noch weiter über eine dipolare 1,3-Cycloaddition eines Azids modifiziert ist, um eine durch Azid-Alkyn modifizierte Nucleobase zu ergeben, wobei das Azid ein lichtempfindliches Molekül umfasst, wobei das lichtempfindliche Molekül eine voluminöse Gruppe ist, die die PCR-Amplifikation des Nucleinsäureliganden hemmt und wobei das lichtempfindliche Molekül einen photospaltbaren Anteil umfasst, wobei, auf das Auslösen der Spaltung des photospaltbaren Anteils durch Strahlung hin, ein verbleibendes kleineres, durch Azid-Alkyn modifiziertes Molekül eine Amplifikation gestattet;
(b) Kontaktierens der Kandidatenmischung mit einer Targetprobe;
(c) Segmentierens von Nucleinsäuren, die sich an die Targetprobe binden, von denjenigen, die sich nicht binden;
(d) Amplifizierens der sich bindenden Nucleinsäuren, um eine Population von Nucleinsäuren herzustellen, die die Targetprobe binden, wodurch ein Aptamer identifiziert oder hergestellt wird.

2. Verfahren nach Anspruch 1, umfassend ferner das Verdrängen eines Strangs mindestens einer Nucleinsäure, die in Schritt (d) amplifiziert worden ist, und das Modifizieren der Nucleinsäure, um eine andere Funktionalisierung einzuführen.

3. Verfahren nach Anspruch 1 oder 2, wobei im Amplifikationsschritt (d) eine Polymerasekettenreaktion unter Verwendung einer durch Alkyn modifizierten Nucleobase angewendet wird, die noch weiter über eine dipolare 1,3-Cycloaddition eines Azids modifiziert ist, um eine durch Azid-Alkyn modifizierte Nucleobase zu ergeben.

4. Verfahren nach Anspruch 1, wobei die Targetprobe aus der Gruppe ausgewählt wird bestehend aus einem Polypeptid, Peptid, einer Zelle, einem Zellfragment, einer Zellmembran, einem kleinen organischen Molekül, einer biologischen Fluidprobe und einer Kombination davon.

5. Reagens umfassend einen Nucleinsäureliganden, der in der Lage ist, eine Targetprobe zu binden, wobei der Nucleinsäureligand mindestens eine Nucleobase umfasst, die modifiziert worden ist, um eine chemische Azid-AlkynGruppe zu enthalten, wobei die chemische Azid-Alkyngruppe ein lichtempfindliches Molekül umfasst, wobei das lichtempfindliche Molekül eine voluminöse Gruppe ist, die die PCR-Amplifikation des Nucleinsäureliganden hemmt und wobei das lichtempfindliche Molekül einen photospaltbaren Anteil umfasst, wobei, auf das Auslösen der Spaltung des photospaltbaren Anteils durch Strahlung hin ein verbleibendes kleineres durch Azid-Alkyn modifiziertes Molekül eine Amplifikation gestattet.

## Revendications

1. Procédé d'identification ou de production d'un aptamère comprenant les étapes de :
(a) préparation d'un mélange d'acides nucléiques candidats, le mélange comprenant au moins un nucléotide qui est modifié pour comprendre une fonctionnalisation introduite par chimie click, dans lequel le nucléotide modifié comprend une base azotée modifiée par alcyne qui est en outre modifiée par une 1,3-cycloaddition dipolaire d'un azide pour obtenir une base azotée modifiée par azide-alcyne, dans lequel l'azide comprend une molécule photosensible, la molécule photosensible étant un groupe volumineux inhibant l'amplification par PCR du ligand d'acide nucléique, et dans lequel la molécule photosensible comprend un fragment photoclivable, dans lequel, lors de l'initiation du clivage du fragment photoclivable par irradiation, une molécule modifiée par azide-alcyne restante, plus petite, permet l'amplification ;
(b) mise en contact du mélange candidat avec un échantillon cible ;
(c) séparation des acides nucléiques qui se lient à l'échantillon cible de ceux qui ne se lient pas ;
(d) amplification des acides nucléiques se liant pour produire une population d'acides nucléiques qui se lient à l'échantillon cible, de façon à identifier ou produire un aptamère.

2. Procédé selon la revendication 1 comprenant en outre le déplacement d'un brin d'au moins un acide nucléique amplifié dans l'étape (d), et en outre la modification de l'acide nucléique pour introduire une autre fonctionnalisation.

3. Procédé selon la revendication 1 ou 2, dans lequel l'étape d'amplification (d) utilise une réaction de polymérase en chaîne utilisant une base azotée modifiée par alcyne qui est en outre modifiée par une 1,3-cycloaddition dipolaire d'un azide pour obtenir une base azotée modifiée par azide-alcyne.

4. Procédé selon la revendication 1, dans lequel l'échantillon cible est choisi dans le groupe constitué d'un polypeptide, un peptide, une cellule, un fragment de cellule, une membrane cellulaire, une petite molécule organique, un échantillon de fluide biologique et une combinaison de ceux-ci.

5. Réactif comprenant un ligand d'acide nucléique capable de se lier à un échantillon cible, dans lequel le ligand d'acide nucléique comprend au moins une base azotée modifiée de façon à contenir un groupe chimique azide-alcyne, dans lequel le groupe chimique azide-alcyne comprend une molécule photosensible, la molécule photosensible étant un groupe volumineux inhibant l'amplification par PCR du ligand d'acide nucléique, et dans lequel la molécule photosensible comprend un fragment photoclivable, dans lequel, lors de l'initiation du clivage du fragment photoclivable par irradiation, une molécule modifiée par azide-alcyne restante, plus petite, permet l'amplification.


### SEQUENCE LISTING

<110> Tolle, Fabian Mayer, Günter Heckel, Alexander Friedrich, Felix
<120> A method of identifying or producing an aptamer
<130> UD 40668/SAM
<150> US 61/885,519
   <151> 2013-10-02
<160> 3
<170> PatentIn version 3.3
<210> 1
   <211> 84
   <212> DNA
   <213> Artificial
<220>
   <223> library
<220>
   <221> misc_feature
   <222> (22)..(63)
   <223> n is a, c, g or u
<400> 1
   cacgacgcaa gggaccacag gnnnnnnnnn nnnnnnnnnn nnnnnnnnnn nnnnnnnnnn        60
   nnncagcacg acaccgcaga ggca                                                84
<210> 2
   <211> 21
   <212> DNA
   <213> Artificial
<220>
   <223> forward primer
<400> 2
   cacgacgcaa gggaccacag g 21
<210> 3
   <211> 21
   <212> DNA
   <213> Artificial
<220>
   <223> reverse primer
<400> 3
   tgcctctgcg gtgtcgtgct g 21